# EUROPEAN PATENT APPLICATION

(11) **EP 4 086 263 A1**
(43) Date of publication of application: **09.11.2022**
(21) Application number: 20910838.0
(22) Date of filing: 07.12.2020
(51) Int. Cl.: C07D 493/02, C07D 495/02, C09K 11/06, H01L 51/50

(54) **ORGANIC ELECTROLUMINESCENCE DEVICE AND AROMATIC COMPOUND CONTAINING FUSED-RING**

(30) Priority: 30.12.2019 CN 201911387703
(71) Applicant: Guangzhou Chinaray Optoelectronic Materials Ltd., Guangzhou, Guangdong 510663 (CN)
(72) Inventor: YANG, Xi, Guangzhou, Guangdong 510663 (CN); CHEN, Jia, Guangzhou, Guangdong 510663 (CN)
(74) Representative: Petraz, Gilberto Luigi
(86) International application number: PCT/CN2020/134314
(87) International publication number: WO 2021/135841

(57) **Abstract**

A compound for an organic electronic device, a mixture, a composition, and the organic electronic device. The compound for the organic electronic device has a structure as shown by general formula (1), has excellent hole transport performance and stability, can be used as a hole injection material of the organic electroluminescence device, and can also be used as a doping agent to be doped in a hole injection layer or a hole transport layer, so that low-voltage driving can be achieved, the electroluminescent efficiency can also be improved, and the service life of the device can be prolonged.

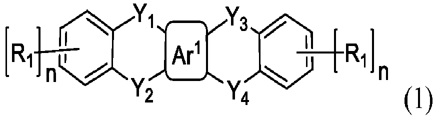

## Description

### FIELD OF INVENTION

The present disclosure relates to the field of organic electroluminescence, and more particularly, to a compound used for an organic electronic device, a mixture, a composition, and the organic electronic device, particularly to an application of the field of electroluminescent devices.

### BACKGROUND OF INVENTION

Organic light-emitting diodes (OLEDs) have advantages of wide variety, low manufacturing costs, and good optical and electrical properties, so they have a great potential for applications in optoelectronic devices (such as flat display panels and lighting).

The organic light-emitting diodes are composed of positive and negative electrodes and an organic layer between the positive and negative electrodes. In order to improve efficiency and service life of the organic light emitting diodes, the organic layer generally has a multi-layer structure, and each layer contains different organic substances. Specifically, the organic layer may include a hole injection layer, a hole transport layer, a light-emitting layer, an electron transport layer, an electron injection layer, etc. A basic principle for the organic light-emitting diodes to emit light is that when a voltage is applied to the two electrodes, the positive electrode injects holes into the organic layer, and the negative electrode injects electrons into the organic layer. When the injected holes meet the electrons, they will form excitons, and the excitons will emit light when they transition back to the ground state. The organic light-emitting diodes have advantages of self-illumination, high brightness, high efficiency, low driving voltages, wide viewing angles, high contrasts, and fast response times. In order to improve recombination efficiency of injected holes and electrons, it is necessary to further improve structures and materials of the organic light-emitting diodes.

In order to obtain high-performance organic light-emitting diodes, injection and high-efficient transport of the holes are the keys. In current OLED light-emitting devices, triarylamine derivatives and carbazole derivatives are usually used as hole injection materials. However, it is still necessary to further improve service life, efficiency, and working voltages thereof.

Therefore, it is still necessary to develop the hole injection materials which can further improve the efficiency and service life of the organic light-emitting diodes.

### SUMMARY OF INVENTION

In view of deficiencies of current technology mentioned above, an objective of the present disclosure is to provide a compound used for an organic electronic device, a mixture, a composition, and the organic electronic device. The objective is to provide a new type of organic optoelectronic functional materials to improve the efficiency and service life of devices.

Technical solutions of the present disclosure are as follows.

A compound used for an organic electronic device, which has a structure as shown by general formula (1): 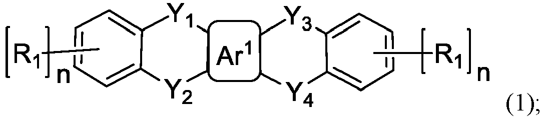 wherein, Ar¹ is selected from structural formulas (A-1) or (A-2): 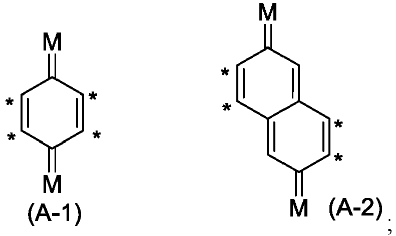
M is selected from CR₂R₃, NR₂, SiR₂R₃, PR₂, a substituted or unsubstituted aromatic group with 6 to 60 carbon atoms, a substituted or unsubstituted heteroaromatic group with 5 to 60 ring atoms, or a non-aromatic ring group with 3 to 30 ring atoms;
Y₁ to Y₄ are each independently selected from a single bond, CR₄R₅, NR₄, O, S, SiR₄R₅, PR₄, P(=O)R₄, S=O, S(=O)₂, or C=O; and Y₁ and Y₂ are not single bonds at a same time, and Y₃ and Y₄ are not single bonds at a same time;
n is an integer ranging from 0 to 4;
R₁ to R₅ are independently selected from H, D, a linear alkyl group, alkoxy group, or thioalkoxy group with 1 to 20 carbon atoms, a branched alkyl group, cyclic alkyl group, alkoxy group, or thioalkoxy group with 3 to 20 carbon atoms, a silyl group, a keto group with 1 to 20 carbon atoms, an alkoxycarbonyl group with 2 to 20 carbon atoms, an aryloxycarbonyl group with 7 to 20 carbon atoms, a cyano group, a carbamoyl group, a haloformyl group, a formyl group, an isocyano group, an isocyanate group, a thiocyanate group, an isothiocyanate group, a hydroxyl group, a nitro group, a nitroso group, CF₃, Cl, Br, F, I, a crosslinkable group, a substituted or unsubstituted aromatic group or heteroaromatic group with 5 to 60 ring atoms, an aryloxy group or heteroaryloxy group with 5 to 60 ring atoms, or a combination thereof; and adjacent R₁ may be bonded to each other to form a substituted or unsubstituted ring; and
* represents a linkage site.

The present disclosure is further related to a mixture, which includes the compound used for the organic electronic device mentioned above and at least one organic functional material, wherein, the organic functional material is a hole injection material, a hole transport material, an electron transport material, an electron injection material, an electron blocking material, a hole blocking material, an emitter, a host material, or an organic dye.

The present disclosure is further related to a composition, which includes the compound used for the organic electronic device mentioned above or the mixture mentioned above, and at least one organic solvent.

The present disclosure is further related to an organic electronic device, which includes at least one functional layer, and a material of the functional layer includes the compound used for the organic electronic device mentioned above or the mixture mentioned above, or is prepared by the composition mentioned above. Preferably, the functional layer is a hole injection layer.

Beneficial effect: the compound used for the organic electronic device in the present disclosure is an indolocarbazole organic compound, which is easily synthesized. When it is used as a P-dopant in the hole injection layer of the organic electronic device, the service life and efficiency of devices can be effectively improved while reducing working voltages.

### DESCRIPTION OF DRAWINGS

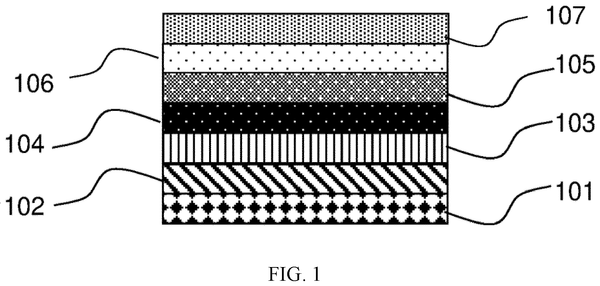

FIG. 1 is a schematic structural diagram of an organic light-emitting device according to an embodiment of the present disclosure. In the figure, 101 denotes a substrate, 102 denotes an anode, 103 denotes a hole injection layer (HIL), 104 denotes a hole transport layer (HTL), 105 denotes a light-emitting layer, 106 denotes an electron injection layer (EIL) or an electron transport layer (ETL), and 107 denotes a cathode.

### DETAILED DESCRIPTION OF PREFERRED EMBODIMENTS

The present disclosure provides a compound used for an organic electronic device, a mixture, a composition, and an organic electronic device. In order to make the purpose, technical solutions, and effects of the present disclosure clearer and more definite, the following further describes the present disclosure in detail. It should be understood that the specific embodiments described herein are only used to explain the disclosure, and are not used to limit the disclosure.

In the present disclosure, "substituted" means that a hydrogen atom in a parent compound is replaced by a substituent.

In the present disclosure, when a same substituent appears multiple times, it can be independently selected from different groups. For example, a general formula has a plurality of M, and M may be independently selected from different groups.

In the present disclosure, "number of ring atoms" means a number of atoms that are bonded to each other and constitute a ring of a structural compound (such as a monocyclic compound, a fused ring compound, a cross-linked compound, a carbocyclic compound, and a heterocyclic compound). When the ring is substituted by a substituent, atoms of the substituent are not included in the ring atoms. The same applies to the "number of ring atoms" described below unless otherwise specified. For example, the number of ring atoms of a benzene ring is 6, the number of ring atoms of a naphthalene ring is 10, and the number of ring atoms of a thienyl group is 5.

An aromatic group refers to a hydrocarbon group containing at least one aromatic ring. A heteroaromatic group refers to an aromatic hydrocarbon group containing at least one heteroatom. The heteroatom is preferably selected from Si, N, P, O, S, and/or Ge, and more preferably from Si, N, P, O, and/or S. A fused cyclic aromatic group means that an aromatic group has two or more rings, in which two carbon atoms are shared by two adjacent rings, that is, a fused ring. A fused heterocyclic aromatic group refers to a fused cyclic aromatic hydrocarbon group containing at least one heteroatom. For the objective of the present disclosure, an aromatic group or heteroaromatic group includes not only aromatic ring systems, but also non-aromatic ring systems. Therefore, for the objective of the present disclosure, similarly, systems, such as pyridine, thiophene, pyrrole, pyrazole, triazole, imidazole, oxazole, oxadiazole, thiazole, tetrazole, pyrazine, pyridazine, pyrimidine, triazine, and carbene, are also considered as an aromatic group or a heterocyclic aromatic group. For the objective of the present disclosure, fused cyclic aromatic groups or fused cyclic heteroaromatic groups include not only systems of aromatic groups or heteroaromatic groups, but also groups in which a plurality of aromatic groups or heteroaromatic groups may also be interrupted by short non-aromatic units (less than 10% of non-hydrogen atoms, preferably less than 5% of non-hydrogen atoms, such as C, N, or O). Therefore, systems such as 9,9'-spirobifluorene, 9,9-diarylfluorene, triarylamine, diarylether, etc., are also considered to be fused cyclic aromatic systems for the objective of the present disclosure.

The present disclosure is related to a compound used for an organic electronic device, which has a structure as shown by general formula (1): 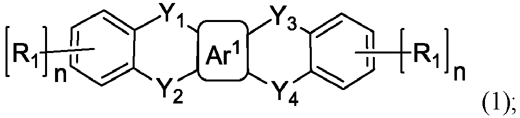 wherein, Ar¹ is selected from structural formulas (A-1) or (A-2): 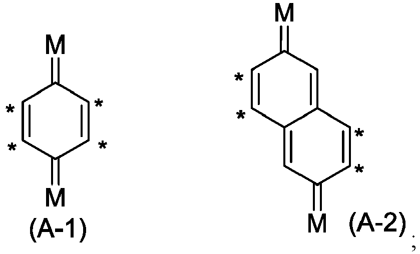
M is selected from CR₂R₃, NR₂, SiR₂R₃, PR₂, a substituted or unsubstituted aromatic group with 6 to 60 carbon atoms, a substituted or unsubstituted heteroaromatic group with 5 to 60 ring atoms, or a non-aromatic ring group with 3 to 30 ring atoms;
Y₁ to Y₄ are each independently selected from a single bond, CR₄R₅, NR₄, O, S, SiR₄R₅, PR₄, P(=O)R₄, S=O, S(=O)₂, or C=O; and Y₁ and Y₂ are not single bonds at a same time, and Y₃ and Y₄ are not single bonds at a same time;
n is an integer ranging from 0 to 4;
R₁ to R₅ are independently selected from H, D, a linear alkyl group, alkoxy group, or thioalkoxy group with 1 to 20 carbon atoms, a branched alkyl group, cyclic alkyl group, alkoxy group, or thioalkoxy group with 3 to 20 carbon atoms, a silyl group, a keto group with 1 to 20 carbon atoms, an alkoxycarbonyl group with 2 to 20 carbon atoms, an aryloxycarbonyl group with 7 to 20 carbon atoms, a cyano group, a carbamoyl group, a haloformyl group, a formyl group, an isocyano group, an isocyanate group, a thiocyanate group, an isothiocyanate group, a hydroxyl group, a nitro group, a nitroso group, CF₃, Cl, Br, F, I, a crosslinkable group, a substituted or unsubstituted aromatic group or heteroaromatic group with 5 to 60 ring atoms, an aryloxy group or heteroaryloxy group with 5 to 60 ring atoms, or a combination thereof; and adjacent R1 may be bonded to each other to form a substituted or unsubstituted ring; and
^{∗} represents a linkage site.

In an embodiment, Ar¹ is the structural formula (A-1), and the general formula (1) is one selected from following general formula:

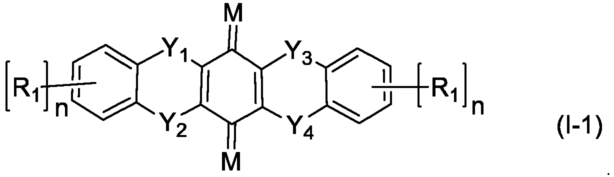

Further, general formula (I-1) is one selected from following general formulas:

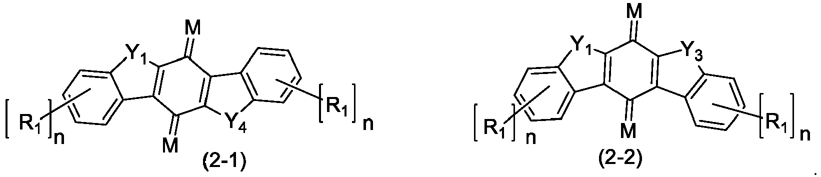

In an embodiment, in general formula (I-1), general formula (2-1), or general formula (2-2), n is an integer ranging from 1 to 4.

In an embodiment, Ar¹ is the structural formula (A-2), and the general formula (1) is one selected from following general formulas:

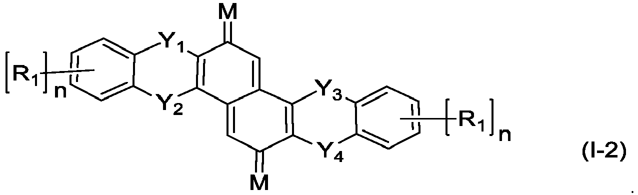

Further, general formula (2-1) is one selected from following general formulas:

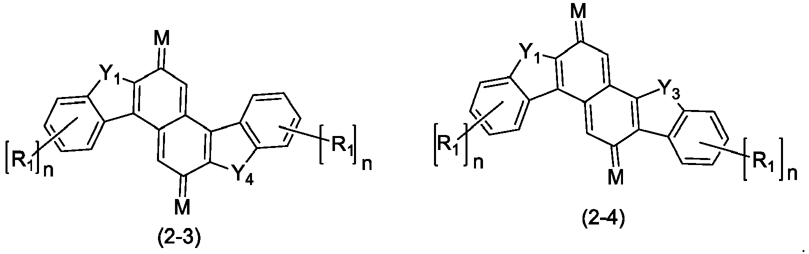

In an embodiment, in general formula (1-2), general formula (2-3), or general formula (2-4), n is 0.

In an embodiment, in general formula (1-2), general formula (2-3), or general formula (2-4), n is an integer ranging from 1 to 4.

In an embodiment, R₁ is each independently selected from D, a linear alkyl group with 1 to 20 carbon atoms, a branched or cyclic alkyl group with 3 to 20 carbon atoms, a cyano group, a carbamoyl group, a haloformyl group, a formyl group, an isocyano group, an isocyanate group, a thiocyanate group, an isothiocyanate group, a hydroxyl group, a nitro group, CF₃, Cl, Br, F, I, a substituted or unsubstituted aromatic group or heteroaromatic group with 5 to 60 ring atoms, or a combination thereof; and adjacent R₁ may be bonded to each other to form a substituted or unsubstituted ring.

In an embodiment, each R₁ is independently selected from a cyano group, a nitro group, a nitroso group, CF₃, Cl, Br, I, F, or an aromatic group or heteroaromatic group substituted with a cyano group, a nitro group, a nitroso group, CF₃, Cl, Br, I, or F.

In an embodiment, each R₁ is one selected from a same group.

In an embodiment, R₁ is one selected from an aromatic group or heteroaromatic group substituted with a cyano group, a nitro group, a nitroso group, CF₃, Cl, Br, I, or F.

Further, in general formula (I-1), general formula (2-1), or general formula (2-2), R₁ is one selected from an aromatic group or heteroaromatic group substituted with a cyano group, a nitro group, a nitroso group, CF₃, Cl, Br, I, or F; further, in general formula (I-1), general formula (2-1), or general formula (2-2), R₁ is one selected from an aromatic group or heteroaromatic group having a number of ring atoms being 6, and substituted with a cyano group, a nitro group, a nitroso group, CF₃, Cl, Br, I, or F.

In an embodiment, Y₁ to Y₄ are independently selected from a single bond, CR₄R₅, O, or S.

In an embodiment, Y₁ and Y₄ are a same group, and preferably, Y₁ and Y₄ are O, S, or CR₄R₅ at a same time.

In an embodiment, Y₁ and Y₃ are a same group, and preferably, Y₁ and Y₃ are O, S, or CR₄R₅ at a same time.

In an embodiment, in the general formula (2-4), Y₁ and Y₃ are S.

In a preferred embodiment, according to the compound used for the organic electronic device, when M appears multiple times, each M is independently selected from CR₂R₃ or NR₂.

In an embodiment, according to the compound used for the organic electronic device, when M appears multiple times, each M is independently selected from CR₂R₃. Preferably, R₂ and R₃ are independently selected from a cyano group, a nitro group, a nitroso group, CF₃, Cl, Br, I, F, or an aromatic group or heteroaromatic group substituted with a cyano group, a nitro group, a nitroso group, CF₃, Cl, Br, I, or F.

In a preferred embodiment, M is one selected from following groups: 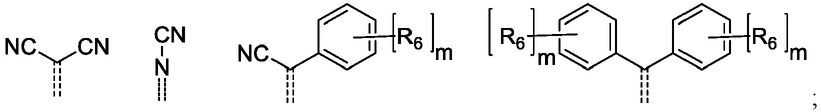
wherein, each R₆ is independently selected from a cyano group, a nitro group, a nitroso group, CF₃, Cl, Br, I, or F; and
m is an integer ranging from 0 to 5, and preferably, m is an integer ranging from 1 to 5.

Further, M is one selected from following groups:

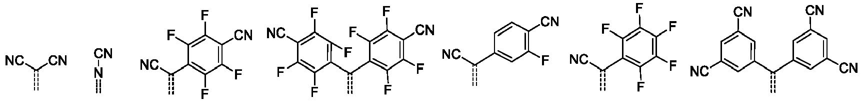

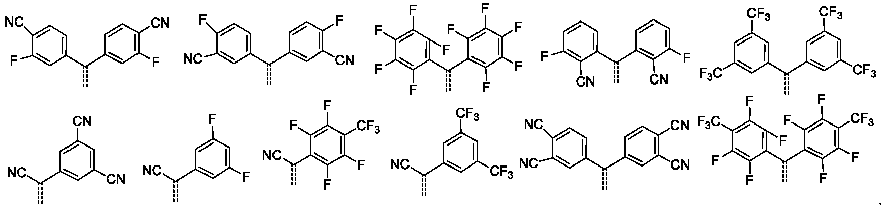

In an embodiment, each M is one selected from a same group; further, M is one selected from

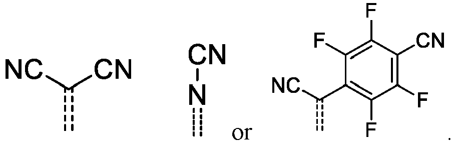

In an embodiment, preferably, the aromatic group or heteroaromatic group substituted with the cyano group, the nitro group, the nitroso group, CF₃, Cl, Br, I, or F is one selected from following groups: 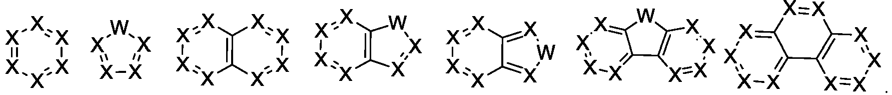 wherein, each X is independently selected from CR₇ or N;
each W is independently selected from CR₈R₉, NR₈, O, S, SiR₈R₉, PR₈, P(=O)R₈, S=O, S(=O)₂, or C=O; and
R₇ to R₉ are each independently selected from H, D, a linear alkyl group with 1 to 20 carbon atoms, a branched or cyclic alkyl group with 3 to 20 carbon atoms, a keto group with 1 to 20 carbon atoms, an alkoxycarbonyl group with 2 to 20 carbon atoms, an aryloxycarbonyl group with 7 to 20 carbon atoms, a cyano group, a carbamoyl group, a cyano group, a nitro group, a nitroso group, CF₃, Cl, Br, I, F, a substituted or unsubstituted aromatic or heteroaromatic group with 5 to 60 ring atoms, an aryloxy group or heteroaryloxy group with 5 to 60 ring atoms, or a combination thereof; and at least one R7 is selected from a cyano group, a nitro group, a nitroso group, CF₃, Cl, Br, I, or F.

In an embodiment, each R₁ is independently selected from 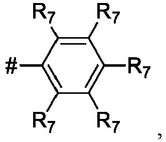 wherein, preferably, each R7 is independently selected from H, D, a cyano group, a nitro group, a nitroso group, CF₃, Cl, Br, I, or F, and at least one R₇ is selected from a cyano group, a nitro group, a nitroso group, CF3, Cl, Br, I, or F.

Further, the aromatic group or heteroaromatic group substituted with the cyano group, the nitro group, the nitroso group, CF₃, Cl, Br, I, or F is preferably one selected from following groups:

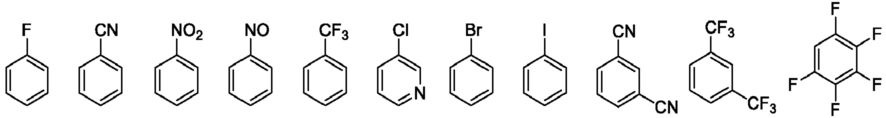

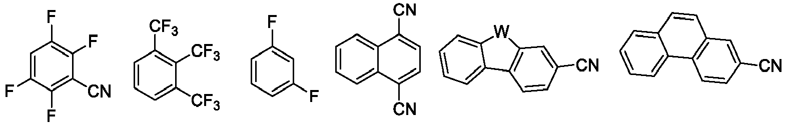

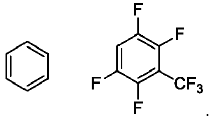

In a preferred embodiment, according to the compound used for the organic electronic device, the general formula (1) is preferably selected from general formulas (1-3) or (1-4):

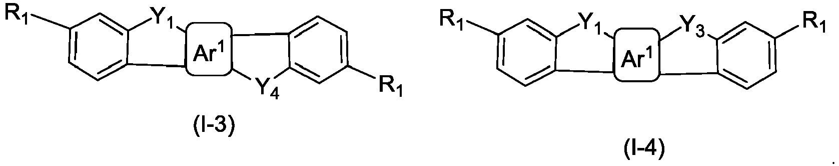

Further, in the (1-3) or (1-4), R₁ is one selected from an aromatic group or heteroaromatic group substituted with a cyano group, a nitro group, a nitroso group, CF₃, Cl, Br, I, or F.

Further, the general formula (1) is preferably selected from general formulas (1-5) or (I-6): 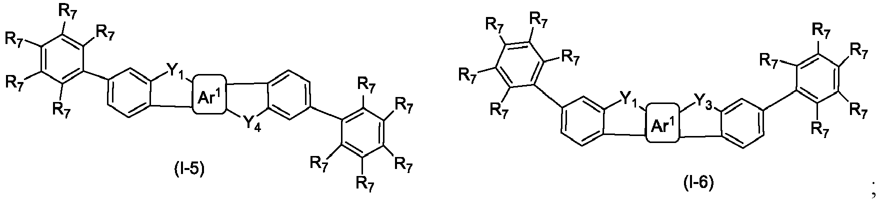 wherein, the definition of R₇ is same as above, and preferably, when R₇ appears multiple times, each R7 is independently selected from a cyano group, a nitro group, a nitroso group, CF₃, Cl, Br, I, or F.

In an embodiment, the compound used for the organic electronic device is further limited to be a material of a hole injection layer of the organic electronic device or a p-dopant material of a hole transport layer of the organic electronic device.

Possible structures of the compound used for the organic electronic device in the present disclosure are listed in the following, but are not limited to these:

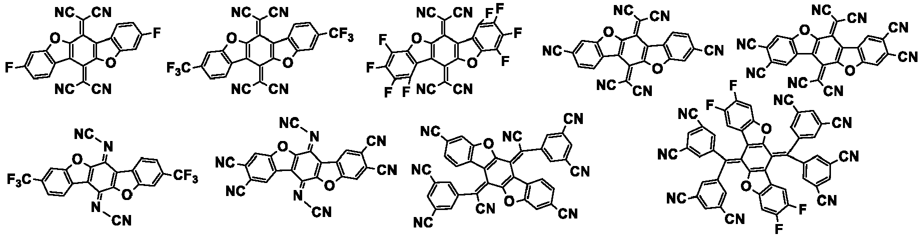

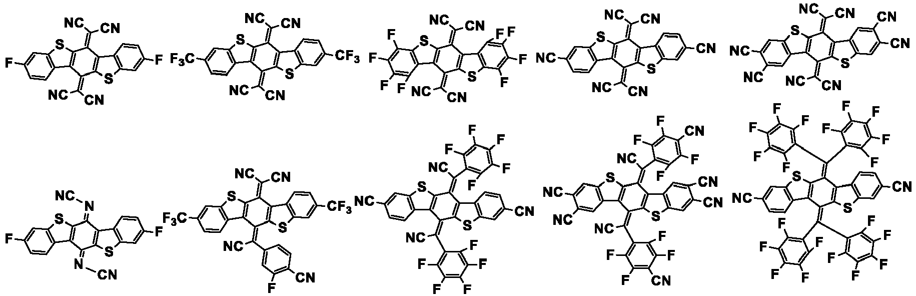

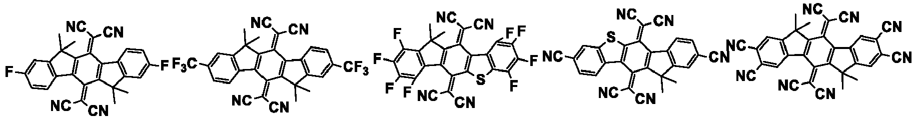

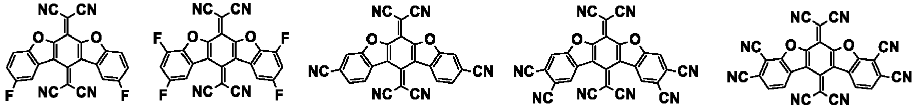

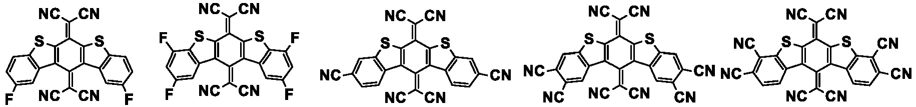

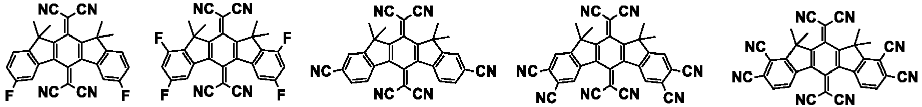

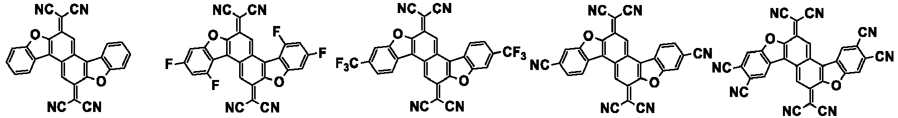

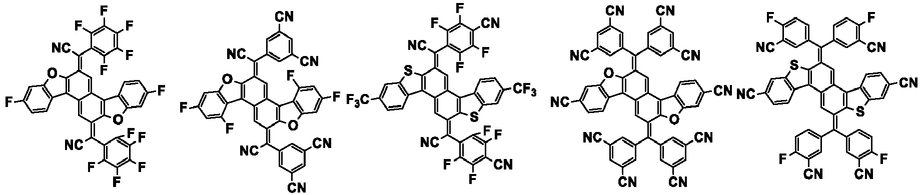

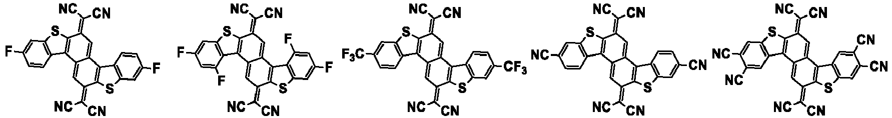

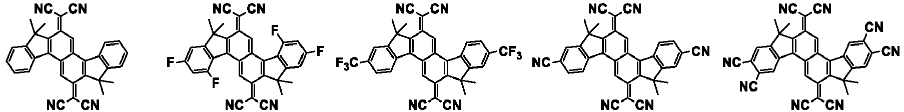

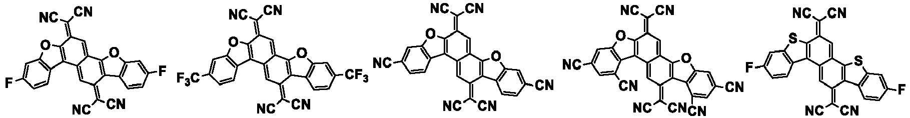

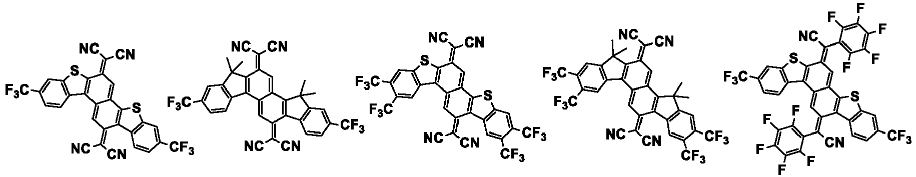

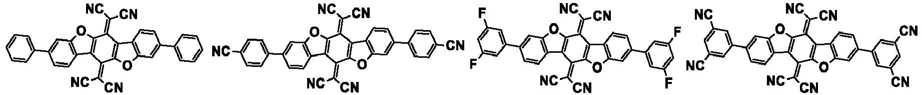

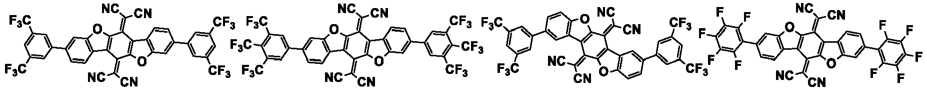

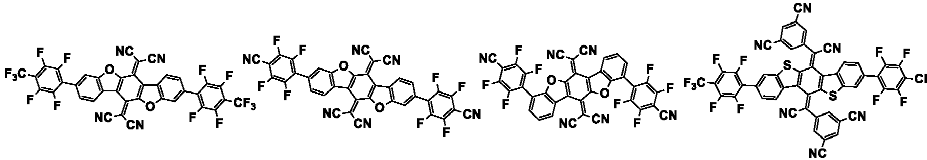

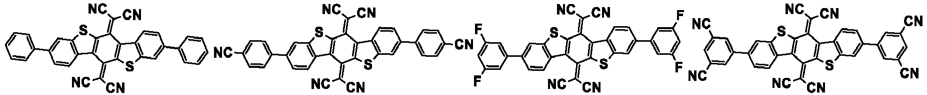

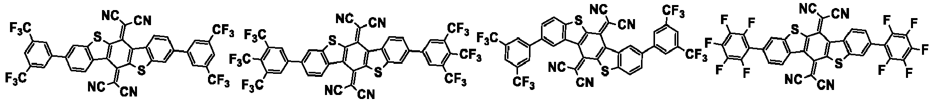

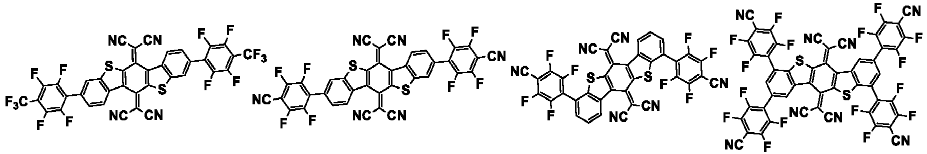

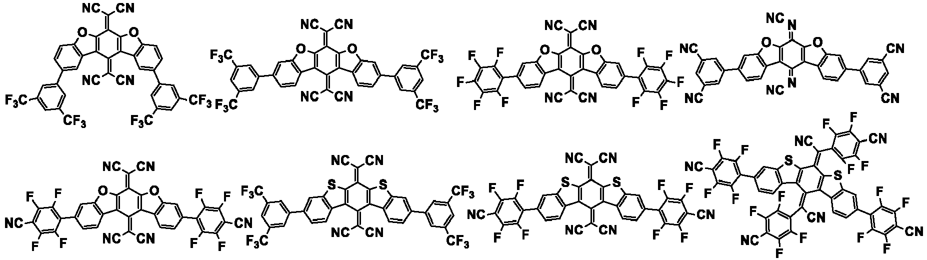

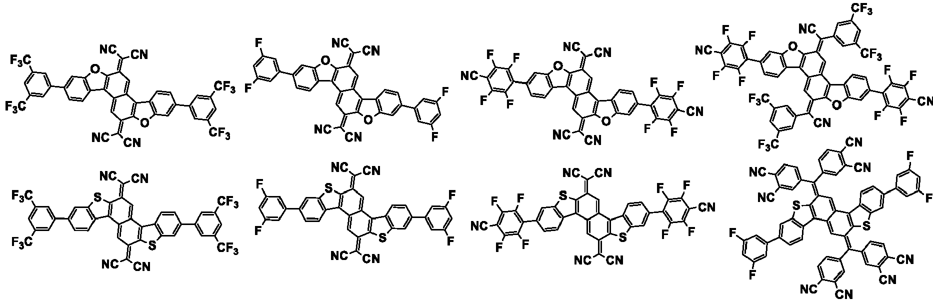

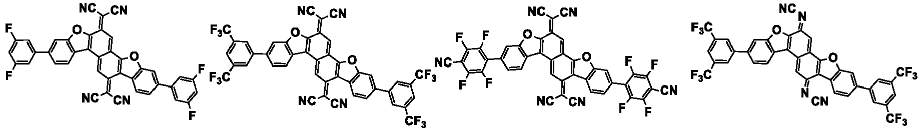

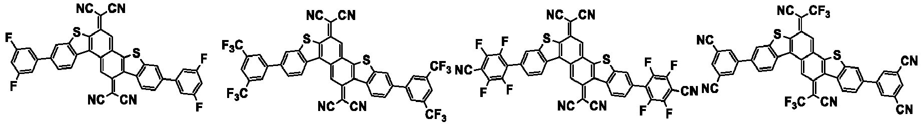

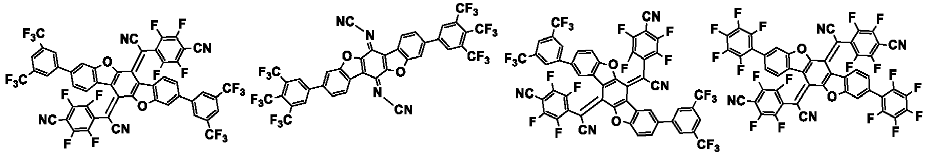

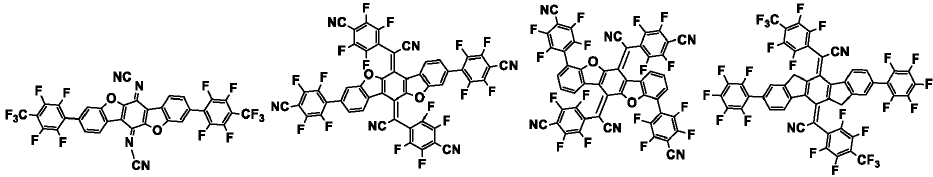

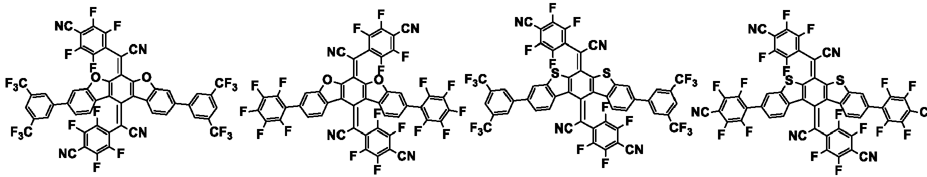

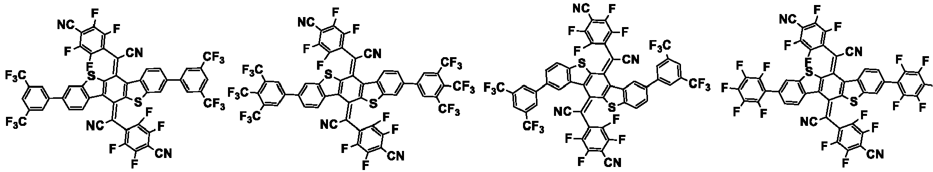

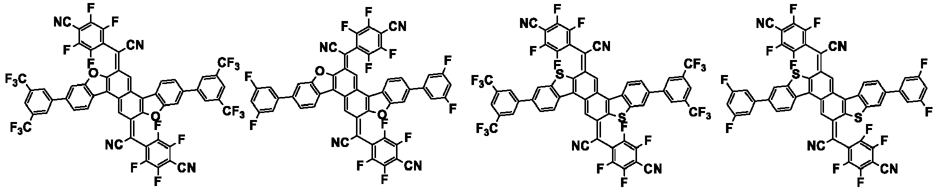

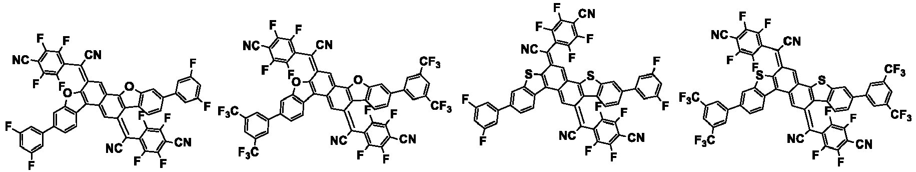

The compound used for the organic electronic device in the present disclosure may be used as a functional material and applied to the functional layer of the organic electronic device. Organic functional layers include, but are not limited to, a hole injection layer (HIL), a hole transport layer (HTL), an electron transport layer (ETL), an electron injection layer (EIL), an electron blocking layer (EBL), a hole blocking layer (HBL), and a light-emitting layer (EML).

In a preferred embodiment, the compound of the present disclosure is used in the hole injection layer or used as a P-dopant.

The present disclosure further provides a mixture, which includes the compound used for the organic electronic device mentioned above and at least one organic functional material. Wherein, the organic functional material may be the hole injection material (HIM), the hole transport material (HTM), the electron transport material (ETM), the electron injection material (EIM), the electron blocking material (EBM), the hole blocking material (HBM), a light-emitting material (an emitter), a host material (Host), or an organic dye. For example, various organic functional materials are described in detail in WO 2010135519 A1, US 20090134784 A1, and WO 2011110277 A1, and the entire contents of these three patent documents are hereby incorporated by reference.

In a preferred embodiment, in the mixture, the at least one organic functional material may be the hole injection material (HIM), the hole transport material (HTM), or the host material (Host).

In an embodiment, the mixture includes at least one hole injection material (HIM) or hole transport material, and a dopant. The dopant is the compound used for the organic electronic device, and a molar ratio of the dopant to a host ranges from 1:1 to 1: 100000.

The detailed description of HIM/HTM/EBM and the Host (the host material/matrix material) can refer to the patent WO2018095395 A1.

Another objective of the present disclosure is to provide material solutions for printing OLEDs.

In some embodiments, the compound of the present disclosure has a molecular weight greater than or equal to 800 g/mol, preferably greater than or equal to 900 g/mol, more preferably greater than or equal to 1000 g/mol, even more preferably greater than or equal to 1100 g/mol, and most preferably greater than or equal to 1200 g/mol.

In some embodiments, the compound of the present disclosure at 25°C has a solubility in toluene greater than or equal to 2 mg/ml, preferably greater than or equal to 3 mg/ml, more preferably greater than or equal to 4 mg/ml, and even more preferably greater than or equal to 5 mg/ml.

The present disclosure is further related to a composition, which includes at least one of the compounds used for the organic electronic device mentioned above or the mixture mentioned above, and at least one organic solvent. The at least one organic solvent is one selected from aromatics, heteroaromatics, esters, aromatic ketones, aromatic ethers, aliphatic ketones, aliphatic ethers, alicyclic or olefin compounds, boronates, or phosphate ester compounds, or is a mixture of two or more than two solvents.

In a preferred embodiment, in the composition of the present disclosure, the at least one organic solvent is selected from aromatic or heteroaromatic based solvents.

Examples of the aromatic or heteroaromatic based solvents that are suitable for the present disclosure are as follows, but are not limited to: p-diisopropylbenzene, pentylbenzene, tetrahydronaphthalene, cyclohexylbenzene, chloronaphthalene, 1,4-dimethylnaphthalene, 3-isopropylbiphenyl, p-methylcumene, dipentylbenzene, tripentylbenzene, amyltoluene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, p-diisopropylbenzene, cyclohexylbenzene, benzylbutylbenzene, dimethylnaphthalene, 3-isopropyl biphenyl, p-methylcumene, 1-methylnaphthalene, 1,2,4-trichlorobenzene, 4,4-difluorodiphenylmethane, 1,2-dimethoxy-4-(1-propenyl)benzene, diphenylmethane, 2-phenyl pyridine, 3-phenyl pyridine, N-methyl diphenylamine, 4-isopropyl biphenyl, α, α-dichlorodiphenylmethane, 4-(3-phenylpropyl)pyridine, benzyl benzoate, 1,1-bis(3,4-dimethylphenyl)ethane, 2-isopropylnaphthalene, quinoline, isoquinoline, methyl 2-furoate, and ethyl 2-furancarboxylate.

Examples of the aromatic ketone based solvents that are suitable for the present disclosure are as follows, but are not limited to: 1-tetralone, 2-tetralone, 2-(phenylepoxy)tetralone, 6-(methoxy)tetralone, acetophenone, propiophenone, benzophenone , and their derivatives, such as 4-methylacetophenone, 3-methylacetophenone, 2-methylacetophenone, 4-methylpropiophenone, 3-methylpropiophenone, and 2-methylpropiophenone.

Examples of the aromatic ether based solvents that are suitable for the present disclosure are as follows, but are not limited to: 3-phenoxytoluene, butoxybenzene, p-anisaldehyde dimethylacetal, tetrahydro-2-phenoxy-2H-pyran, 1,2-dimethoxy-4-(1-propenyl) benzene, 1,4-benzodioxane, 1,3-dipropylbenzene, 2,5-dimethoxytoluene, 4-ethyl phenethyl ether, 1,3-dipropoxybenzene, 1,2,4-trimethoxybenzene, 4-(1 -propenyl)-1,2-dimethoxybenzene, 1,3-dimethoxybenzene, glycidyl phenyl ether, dibenzyl ether, 4-tert-butyl anisole, trans-p-propenyl anisole, 1,2-dimethoxybenzene, 1-methoxynaphthalene, diphenyl ether, 2-phenoxymethyl ether, 2- phenoxytetrahydrofuran, and ethyl-2-naphthyl ether.

In some preferred embodiments, in the composition of the present disclosure, the at least one organic solvent may be selected from aliphatic ketones, for example, 2-nonanone, 3-nonanone, 5-nonanone, 2-decanone, 2,5-hexanedione, 2,6,8-trimethyl-4-nonanone, fenone, phorone, isophorone, and di-n-amyl ketone, or aliphatic ethers, for example, amyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethylene glycol ethyl methyl ether, triethylene glycol butyl methyl ether, tripropylene glycol dimethyl ether, and tetraethylene glycol dimethyl ether.

In some preferred embodiments, in the composition of the present disclosure, the at least one organic solvent may be selected from ester-based solvents: alkyl octanoate, alkyl sebacate, alkyl stearate, alkyl benzoate, alkyl phenylacetate, alkyl cinnamate, alkyl oxalate, alkyl maleate, alkyl lactone, and alkyl oleate. Preferably, the at least one organic solvent may be octyl octanoate, diethyl sebacate, diallyl phthalate, or isononyl isononanoate.

The solvents mentioned above may be used alone or as a mixture of two or more organic solvents.

In some preferred embodiments, the composition of the present disclosure may include at least one of the compounds mentioned above, a polymer, or the mixture, and the at least one organic solvent, and may further include another organic solvent. Examples of another organic solvent includes but is not limited to: methanol, ethanol, 2-methoxyethanol, dichloromethane, chloroform, chlorobenzene, o-dichlorobenzene, tetrahydrofuran, anisole, morpholine, toluene, o-xylene, m-xylene, p-xylene, 1,4-dioxane, acetone, methyl ethyl ketone, 1,2 dichloroethane, 3-phenoxytoluene, 1,1,1-trichloroethane, 1,1,2,2-tetrachloroethane, ethyl acetate, butyl acetate, dimethylformamide, dimethylacetamide, dimethylsulfoxide, tetrahydronaphthalene, decalin, indene, and/or a mixture thereof.

In some preferred embodiments, solvents that are particularly suitable for the composition of the present disclosure are those having a Hansen solubility parameter in following ranges:
δ_{d} (dispersion force) in a range of 17.0 to 23.2 MPa^{1/2}, preferably 18.5 to 21.0 MPa^{1/2};
δₚ (polar force) in a range of 0.2 to 12.5 MPa^{1/2}, preferably 2.0 to 6.0 MPa^{1/2}; and
δₕ (hydrogen bonding force) in a range of 0.9 to 14.2 MPa^{1/2}, preferably 2.0 to 6.0 MPa^{1/2}.

In the composition of the present disclosure, a boiling point parameter of the organic solvent should be considered when selecting. In the present disclosure, the boiling point of the organic solvent is greater than or equal to 150°C, preferably greater than or equal to 180°C, more preferably greater than or equal to 200°C, more preferably greater than or equal to 250°C, and even more preferably greater than or equal to 275°C or 300°C. The boiling point in these ranges is beneficial for preventing nozzle clogging in inkjet printheads. The organic solvent can be evaporated from a solvent system to form a functional material thin film.

In a preferred embodiment, the composition of the present disclosure is a solution.

In another preferred embodiment, the composition of the present disclosure is a suspension.

The composition of the present disclosure may include 0.01 to 10 wt% of the compound used for the organic electronic device or the mixture, preferably 0.1 to 15 wt%, more preferably 0.2 to 5 wt%, and even more preferably 0.25 to 3 wt%.

The present disclosure is also related to a use of the composition as a coating or printing ink when preparing the organic electronic device by printing or coating.

Suitable printing or coating techniques include, but are not limited to, inkjet printing, nozzle printing, typography, screen printing, dip coating, spin coating, knife coating, roll printing, twist roll printing, planographic printing, flexographic printing, rotary printing, spraying coating, brushing, pad printing, slot extrusion coating, etc. Preferred are gravure printing, nozzle printing, and inkjet printing. The solution or suspension may additionally include one or more components such as surfactants, lubricants, wetting agents, dispersing agents, hydrophobic agents, binders, etc., to adjust viscosity, film-forming properties, improve adhesion, and the like. They are related to printing technology and related requirements for solutions, such as solvents, concentrations, viscosity, etc.

The present disclosure further provides an application of the compound, mixture, or composition in the organic electronic device. The organic electronic device may be, but is not limited to, organic-light emitting diodes (OLEDs), organic photovoltaic cells (OPV), organic light-emitting cells (OLEEC), organic field effect transistors (OFET), organic light-emitting field effect transistors, organic lasers, organic spintronic device, organic sensors, and organic plasmon-emitting diodes, etc., and is preferably OLEDs. In the embodiment of the present disclosure, the compound or the mixture is preferably used in the hole injection layer of OLED devices.

The present disclosure is further related to an organic electronic device, which includes at least one of the compounds used for the organic electronic device mentioned above or the mixture mentioned above, or is prepared by the composition mentioned above. Further, the organic electronic device includes at least one functional layer, and the functional layer includes the compound used for the organic electronic device mentioned above, the mixture mentioned above, or a polymer thereof, or is prepared by the composition mentioned above. The functional layer may be a hole injection layer, a hole transport layer, a light-emitting layer, an electron blocking layer, an electron injection layer, an electron transport layer, or a hole blocking layer.

In a preferred embodiment, the organic electronic device of the present disclosure at least includes the hole injection layer or the hole transport layer, and the hole injection layer or the hole transport layer includes the compound mentioned above.

In general, the organic electronic device of the present disclosure at least includes a cathode, an anode, and the functional layer disposed between the cathode and the anode, and the functional layer at least includes one of the compounds used for the organic electronic device mentioned above. The organic electronic device may be, but is not limited to, organic-light emitting diodes, organic photovoltaic cells, organic light-emitting cells, organic field effect transistors, organic light-emitting field effect transistors, organic lasers, organic spintronic devices, organic sensors, and organic plasmon-emitting diodes, etc., and is preferably organic electroluminescent devices, such as OLEDs, OLEECs, and organic light-emitting field effect transistors.

In some preferred embodiments, in the electroluminescent devices, a hole injection layer or a hole transport layer thereof includes one of the compounds used for the organic electronic device mentioned above.

The light-emitting devices, particularly the OLEDs, include a substrate, an anode, at least one light-emitting layer, and a cathode.

The substrate may be opaque or transparent. A transparent substrate may be used to fabricate a transparent light-emitting device. For example, refer to Bulovic et al., Nature 1996, 380, p29, and Gu et al., Appl. Phys. Lett. 1996, 68, p2606. The substrate may be rigid or flexible. The substrate may be plastics, metals, semiconductor wafers, or glass. Preferably, the substrate has a smooth surface. Substrates free of surface defects are particularly desirable. In a preferred embodiment, the substrate is flexible and may be made of polymer film or plastics, A glass transition temperature Tg is greater than or equal to 150°C, preferably greater than 200°C, more preferably greater than 250°C, and even more preferably greater than 300°C. Examples of suitable flexible substrates are polyethylene terephthalate (PET) and polyethylene glycol(2,6-naphthalene) (PEN).

The anode may include a conductive metal or metal oxide, or a conductive polymer. The anode can easily inject holes into a hole injection layer (HIL), a hole transport layer (HTL), or a light-emitting layer. In an embodiment, an absolute value of a difference between a work function of the anode and a HOMO energy level or valence band energy level of an emitter in the light-emitting layer or a p-type semiconductor material as an HIL, HTL, or electron blocking layer (EBL) is less than 0.5 eV, preferably less than 0.3 eV, and more preferably less than 0.2 eV. Examples of anode materials include, but are not limited to, Al, Cu, Au, Ag, Mg, Fe, Co, Ni, Mn, Pd, Pt, ITO, aluminum-doped zinc oxide (AZO), etc. Other suitable anode materials are known and can be readily selected for use by those of ordinary skill in the art. The anode materials may be deposited using any suitable techniques, such as a suitable physical vapor deposition method, which includes radio frequency magnetron sputtering, vacuum thermal evaporation, electron beam (e-beam), etc. In some embodiments, the anode is patterned. Patterned ITO conductive substrates are commercially available and can be used to fabricate devices of the present disclosure.

The cathode may include a conductive metal or metal oxide. The cathode can easily inject electrons into an EIL or ETL, or directly into the light-emitting layer. In an embodiment, an absolute value of a difference between a work function of the cathode and a LUMO energy level or conduction band energy level of the emitter in the light-emitting layer or an N-type semiconductor material as an electron injection layer (EIL), an electron transport later (ETL), or a hole blocking layer (HBL) is less than 0.5 eV, preferably less than 0.3 eV, and more preferably less than 0.2 eV. In principle, all materials that can be used as cathodes for OLEDs are possible as cathode materials for the devices of the disclosure. Examples of anode materials include, but are not limited to, Al, Au, Ag, Ca, Ba, Mg, LiF/Al, MgAg alloy, BaF2/Al, Cu, Fe, Co, Ni, Mn, Pd, Pt, ITO, etc. The cathode materials may be deposited using any suitable techniques, such as a suitable physical vapor deposition method, which includes radio frequency magnetron sputtering, vacuum thermal evaporation, electron beam (e-beam), etc.

The OLEDs may include other functional layers, such as a hole injection layer (HIL), a hole transport layer (HTL), an electron blocking layer (EBL), an electron injection layer (EIL), an electron transport layer (ETL), and a hole blocking layer (HBL). Materials suitable for use in these functional layers are described in detail above and in WO 2010135519 A1, US 20090134784 A1, and WO 2011110277 A1, and the entire contents of these three patent documents are hereby incorporated by reference.

The light-emitting devices of the present disclosure has a light-emitting wavelength ranging from 300nm to 1200nm, preferably from 350nm to 1000nm, and more preferably from 400nm to 900nm.

The present disclosure is also related to applications of the light-emitting devices of the present disclosure on various electronic devices, which include, but are not limited to, display devices, lighting devices, light sources, sensors, etc.

The present disclosure will be described below with reference to the preferred embodiments, but is not limited to the following embodiments. It should be understood that the appended claims summarize the scope of the present disclosure. Under the guidance of the inventive concept, those skilled in the art should recognize that certain changes made to the various embodiments of the present disclosure will be covered by the spirit and scope of the claims of the present disclosure.

### SPECIFIC EMBODIMENTS

1. The synthesis methods of the compound of the present disclosure are described below, but the present disclosure is not limited to the following examples.

### Synthesis Example 1: Synthesis of Compound 1

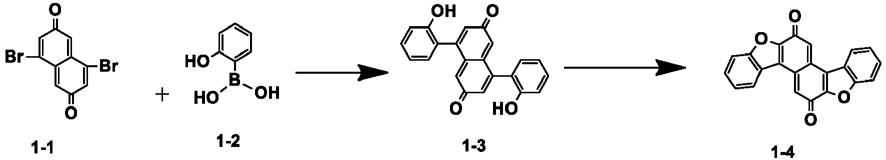

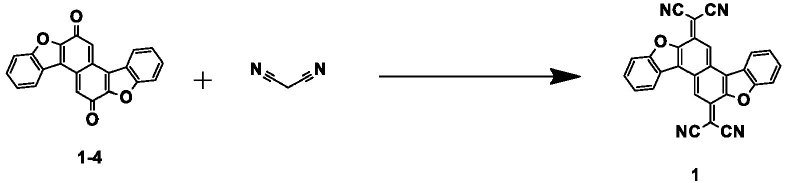

1) Synthesis of intermediate 1-3: under a nitrogen atmosphere, compound 1-1 (6.3 g, 20 mmol), compound 1-2 (5.5 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 1-3. The yield is about 60%.
2) Synthesis of intermediate 1-4: under a nitrogen atmosphere, compound 1-3 (6.8 g, 20 mmol), 20 ml of tert-butyl peroxybenzoate, 30 ml of 3-nitro pyridine, and 100 ml of ethylene glycol dimethyl ether are added to a 250 mL three-necked flask, and reacted at 150°C for 0.5 hours. The reaction solution is washed three times with 150 ml of saturated brine, dried by anhydrous sodium sulfate, and then distilled off under reduced pressure to remove the solvent, and the residue is purified by column using DCM/PE to obtain compound 1-4. The yield is about 50%.
3) Synthesis of compound 1: raw material 1-4 (6.7 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 1 is obtained after washed with water, dried, and purified by column chromatography, and MS:[M+H]+=434.0. The yield is about 40%.

### Synthesis Example 2: Synthesis of Compound 2

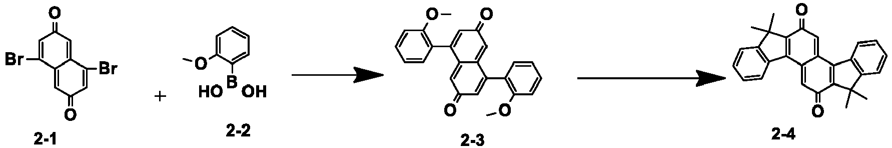

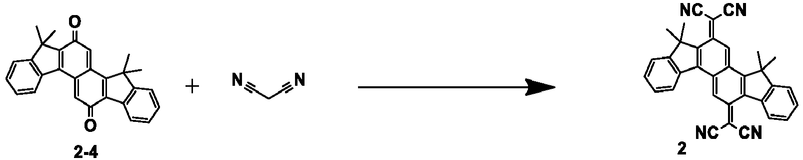

1) Synthesis of intermediate 2-3: under a nitrogen atmosphere, compound 2-1 (6.3 g, 20 mmol), compound 2-2 (6.0 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 2-3. The yield is about 60%.
2) Synthesis of intermediate 2-4: under the nitrogen atmosphere, compound 2-3 (7.4g, 20 mmol) is added into a 250ml two-necked flask, and 50 ml of tetrahydrofuran is added to dissolve the compound 2-3. The temperature is cooled to -78 degrees by liquid nitrogen, and 1M of methylmagnesium bromide (100 ml, 100 mmol) is added to the reaction solution, and then the reaction is carried out at room temperature for 12 hours. The reaction solution is poured into water to quench, washed three times with 150 ml of saturated brine, dried by anhydrous sodium sulfate, and then evaporated to remove the solvent. 30 ml of acetic acid and 30 ml of phosphoric acid are added and stirred for 12 hours. The mixture is poured into ice water, neutralized by adding potassium carbonate, extracted with dichloromethane, and spun dry, and the residue is purified by column using DCM/PE to obtain compound 2-4. The yield is about 40%.
3) Synthesis of compound 2: raw material 2-4 (7.8 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 2 is obtained after washed with water, dried, and purified by column chromatography, and MS:[M+H]+=486.3. The yield is about 40%.

### Synthesis Example 3: Synthesis of Compound 3

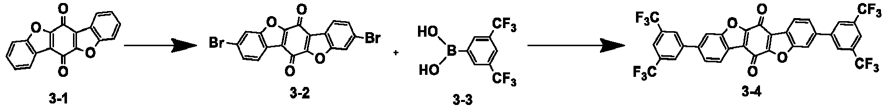

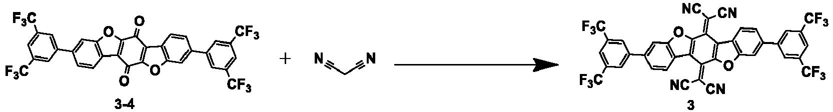

1) Synthesis of intermediate 3-2: compound 3-1 (7.3 g, 20 mmol) is added into a 100 ml two-necked flask, and 40 ml of tetrahydrofuran is added to dissolve the compound 3-1, and then N-bromosuccinimide (7.2 g, 40 mmol) is added and stirred for 12 hours. The reaction solution is then spun dry and recrystallized from dichloromethane and methanol to obtain the intermediate 3-2. The yield is about 45%.
2) Synthesis of intermediate 3-4: under a nitrogen atmosphere, compound 3-2 (8.9 g, 20 mmol), compound 3-3 (10.2 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 3-4. The yield is about 60%.
3) Synthesis of compound 3: raw material 3-4 (14.2 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 3 is obtained after washed with water, dried, and purified by column chromatography, and MS:[M+H]+=808.6. The yield is about 40%.

### Synthesis Example 4: Synthesis of Compound 4

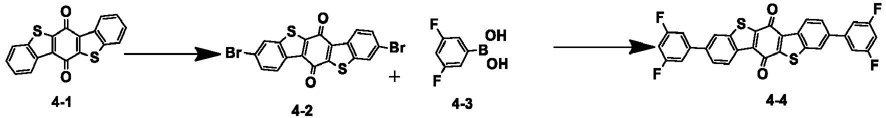

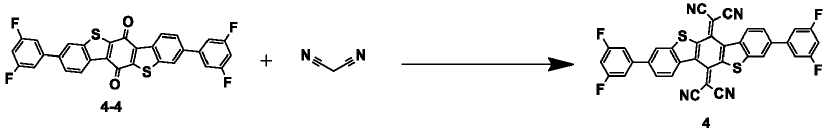

1) Synthesis of intermediate 4-2: compound 4-1 (6.4 g, 20 mmol) is added into a 100 ml two-necked flask, and 40 ml of tetrahydrofuran is added to dissolve the compound 4-1, and then N-bromosuccinimide (7.2 g, 40 mmol) is added and stirred for 12 hours. The reaction solution is then spun dry and recrystallized from dichloromethane and methanol to obtain the intermediate 4-2. The yield is about 45%.
2) Synthesis of intermediate 4-4: under a nitrogen atmosphere, compound 4-2 (9.5 g, 20 mmol), compound 4-3 (6.3 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 4-4. The yield is about 60%.
3) Synthesis of compound 4: raw material 4-4 (10.8 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 4 is obtained after washed with water, dried, and purified by column chromatography, and MS:[M+H]+=640.5. The yield is about 40%.

### Synthesis Example 5: Synthesis of Compound 5

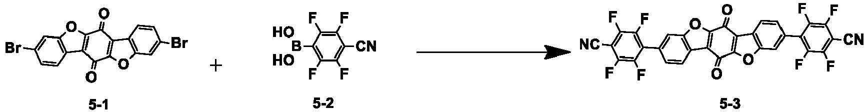

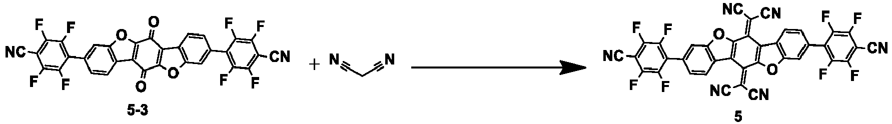

1) Synthesis of intermediate 5-1 is same as the synthesis of compound 3-2.
2) Synthesis of intermediate 5-3: under a nitrogen atmosphere, compound 5-1 (8.8 g, 20 mmol), compound 5-2 (8.7 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 5-3. The yield is about 60%.
3) Synthesis of compound 5: raw material 5-3 (12.6 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 5 is obtained after washed with water, dried, and purified by column chromatography, and MS:[M+H]+=740.1. The yield is about 40%.

### Synthesis Example 6: Synthesis of Compound 6

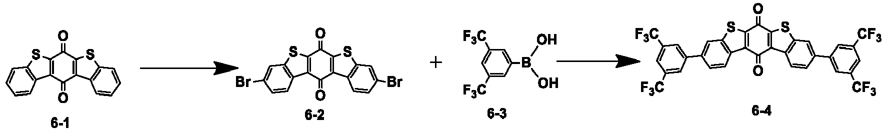

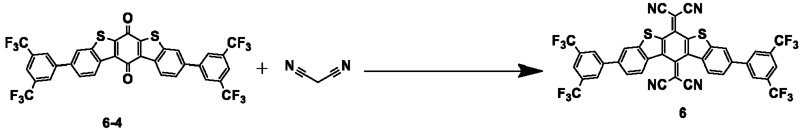

1) Synthesis of intermediate 6-2: compound 6-1 (6.4 g, 20 mmol) is added into a 100 ml two-necked flask, and 40 ml of tetrahydrofuran is added to dissolve the compound 4-1, and then N-bromosuccinimide (7.2 g, 40 mmol) is added and stirred for 12 hours. The reaction solution is then spun dry and recrystallized from dichloromethane and methanol to obtain the intermediate 6-2. The yield is about 45%.
2) Synthesis of intermediate 6-4: under a nitrogen atmosphere, compound 6-2 (9.5 g, 20 mmol), compound 6-3 (10.2 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 6-4. The yield is about 60%.
3) Synthesis of compound 6: raw material 6-4 (14.8 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 6 is obtained after washed with water, dried, and purified by column chromatography, and MS:[M+H]+=840.6. The yield is about 40%.

### Synthesis Example 7: Synthesis of Compound 7

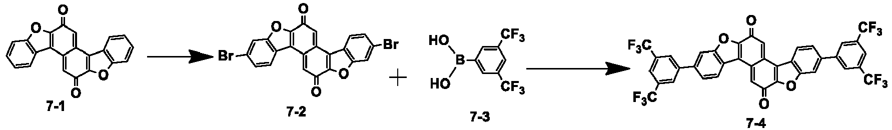

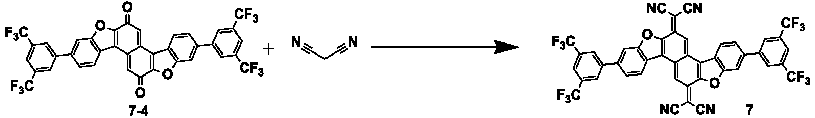

1) Synthesis of intermediate 7-1 is same as the synthesis of compound 1-4.
2) Synthesis of intermediate 7-2: compound 7-1 (6.7 g, 20 mmol) is added into a 100 ml two-necked flask, and 40 ml of tetrahydrofuran is added to dissolve the compound 7-1, and then N-bromosuccinimide (7.2 g, 40 mmol) is added and stirred for 12 hours. The reaction solution is then spun dry and recrystallized from dichloromethane and methanol to obtain the intermediate 7-2. The yield is about 45%.
3) Synthesis of intermediate 7-4: under a nitrogen atmosphere, compound 7-2 (10.0 g, 20 mmol), compound 7-3 (10.2 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 7-4. The yield is about 60%.
4) Synthesis of compound 7: raw material 7-4 (15.2 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 7 is obtained after washed with water, dried, and purified by column chromatography, and MS:[M+H]+=858.4. The yield is about 40%.

### Synthesis Example 8: Synthesis of Compound 8

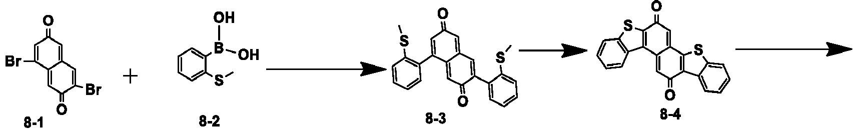

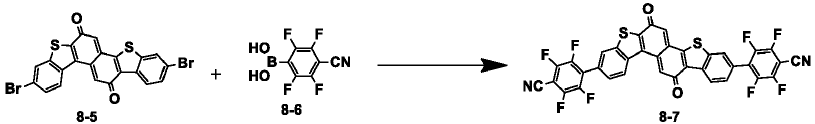

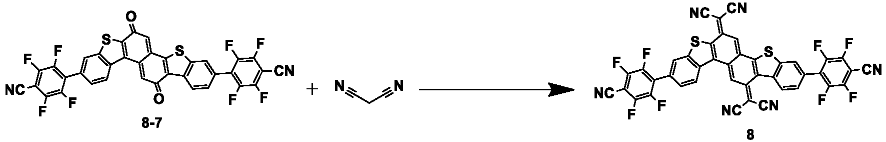

1) Synthesis of intermediate 8-3: under a nitrogen atmosphere, compound 8-1 (6.3 g, 20 mmol), compound 8-2 (6.7 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 8-3. The yield is about 60%.
2) Synthesis of intermediate 8-4: under a nitrogen atmosphere, compound 8-3 (8.0 g, 20 mmol), 20 ml of tert-butyl peroxybenzoate, 30 ml of 3-nitro pyridine, and 100 ml of ethylene glycol dimethyl ether are added to a 250 mL three-necked flask, and reacted at 150°C for 0.5 hours. The reaction solution is washed three times with 150 ml of saturated brine, dried by anhydrous sodium sulfate, and then distilled off under reduced pressure to remove the solvent, and the residue is purified by column using DCM/PE to obtain compound 8-4. The yield is about 50%.
3) Synthesis of intermediate 8-5: compound 8-4 (7.4 g, 20 mmol) is added into a 100 ml two-necked flask, and 40 ml of tetrahydrofuran is added to dissolve the compound 3-1, and then N-bromosuccinimide (7.2 g, 40 mmol) is added and stirred for 12 hours. The reaction solution is then spun dry and recrystallized from dichloromethane and methanol to obtain the intermediate 8-5. The yield is about 45%.
4) Synthesis of intermediate 8-7: under a nitrogen atmosphere, compound 8-5 (10.5 g, 20 mmol), compound 8-6 (8.7 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 8-7. The yield is about 60%.
5) Synthesis of compound 8: raw material 8-7 (14.3 g, 20 mmol) and titanium tetrachloride (0.228 g, 1.2 mmol) are dissolved in 100 ml of chloroform under protection of nitrogen, then malononitrile (3.9 g, 60 mmol) is added under an ice bath, and the reaction solution is stirred overnight at room temperature. The compound 8 is obtained after washed with water, dried, and purified by column chromatography, and MS:[M+H]+=812.6. The yield is about 40%.

### Synthesis Example 9: Synthesis of Compound 9

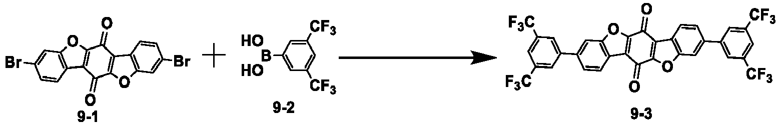

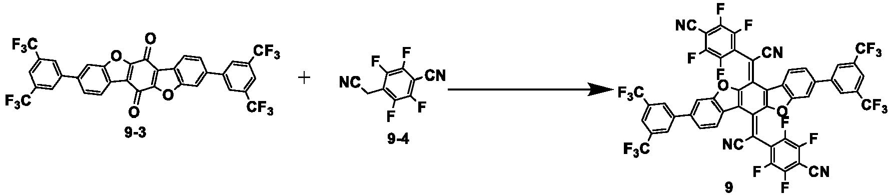

1) Synthesis of intermediate 9-1 is same as the synthesis of compound 3-2.
2) Synthesis of intermediate 9-3: under a nitrogen atmosphere, compound 9-1 (9.0 g, 20 mmol), compound 9-2 (10.2 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 9-3. The yield is about 60%.
3) Synthesis of compound 9: compound 9-3 (14.2 g, 20 mmol) is added into a three-necked flask, then 200 ml of ethanol and 50 ml of 50% aqueous sodium hydroxide are added, and then compound 9-4 (10.7 g, 50 mmol) is added under an ice bath condition. The reaction solution is stirred at room temperature for 12 hours for reaction. TLC and MS shows that the reaction is complete, and the reaction solution is washed three times with 150 ml of saturated brine, extracted by dichloromethane, dried by anhydrous sodium sulfate, and then evaporated to remove the solvent. The residue is purified by column using DCM/PE (1:4) to obtain compound 9, and MS:[M+H]+=1104.8. The yield is about 40%.

### Synthesis Example 10: Synthesis of Compound 10

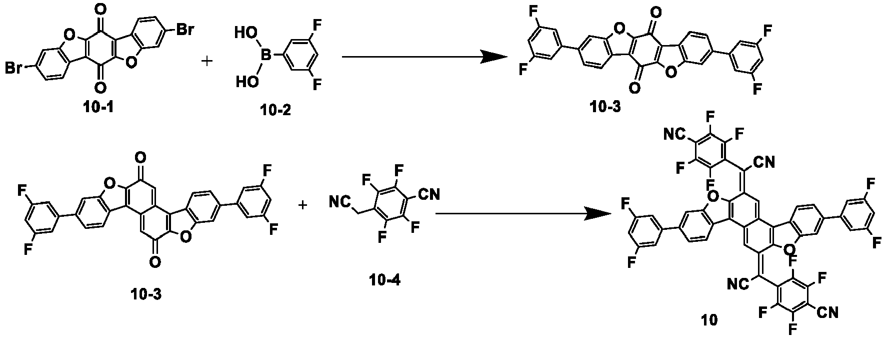

1) Synthesis of intermediate 10-1 is same as the synthesis of compound 3-2.
2) Synthesis of intermediate 10-3: under a nitrogen atmosphere, compound 10-1 (9.0 g, 20 mmol), compound 10-2 (6.3 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 10-3. The yield is about 60%.
3) Synthesis of compound 10: compound 10-3 (11.2 g, 20 mmol) is added into a three-necked flask, then 200 ml of ethanol and 50 ml of 50% aqueous sodium hydroxide are added, and then compound 10-4 (10.7 g, 50 mmol) is added under an ice bath condition. The reaction solution is stirred at room temperature for 12 hours for reaction. TLC and MS shows that the reaction is complete, and the reaction solution is washed three times with 150 ml of saturated brine, extracted by dichloromethane, dried by anhydrous sodium sulfate, and then evaporated to remove the solvent. The residue is purified by column using DCM/PE (1:4) to obtain compound 10, and MS:[M+H]+=954.6. The yield is about 40%.

### Synthesis Example 11: Synthesis of Compound 11

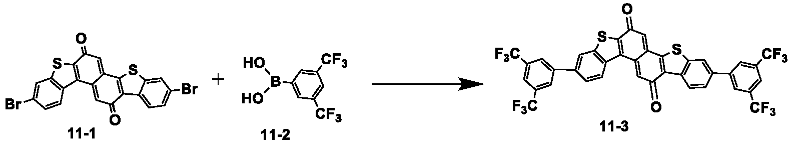

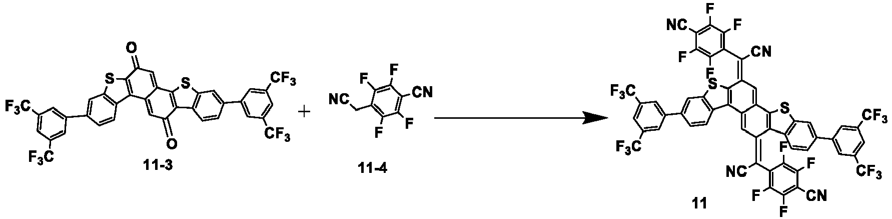

1) Synthesis of intermediate 11-1 is same as the synthesis of compound 8-5.
2) Synthesis of intermediate 11-3: under a nitrogen atmosphere, compound 11-1 (10.5 g, 20 mmol), compound 11-2 (10.2 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 11-3. The yield is about 60%.
3) Synthesis of compound 11: compound 11-3 (13.2 g, 20 mmol) is added into a three-necked flask, then 200 ml of ethanol and 50 ml of 50% aqueous sodium hydroxide are added, and then compound 11-4 (10.7 g, 50 mmol) is added under an ice bath condition. The reaction solution is stirred at room temperature for 12 hours for reaction. TLC and MS shows that the reaction is complete, and the reaction solution is washed three times with 150 ml of saturated brine, extracted by dichloromethane, dried by anhydrous sodium sulfate, and then evaporated to remove the solvent. The residue is purified by column using DCM/PE (1:4) to obtain compound 11, and MS:[M+H]+=1183.1. The yield is about 40%.

### Synthesis Example 12: Synthesis of Compound 12

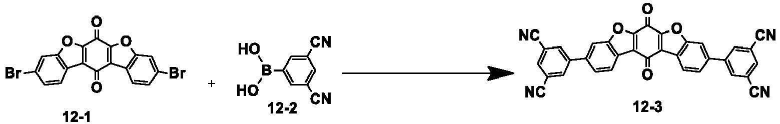

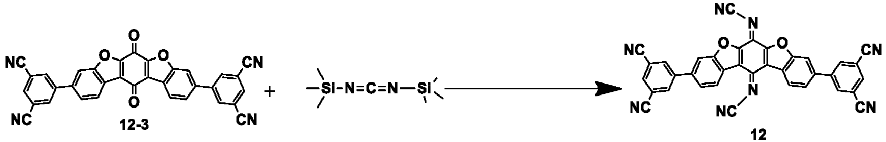

1) Synthesis of intermediate 12-3: under a nitrogen atmosphere, compound 12-1 (8.9 g, 20 mmol), compound 12-2 (6.8 g, 40 mmol), tetrakis(triphenylphosphine)palladium (1.4g, 1.2 mmol), 2.00 mol/L of sodium carbonate (8.48 g, 80 mmol), and toluene (50 mL) are added to a 250 mL three-necked flask, heated at 90°C, and stirred for 12 hours for reaction. When the reaction is completed, TLC and MS shows that the reaction is complete, and mainly the target product is obtained. The flask is cooled, the reaction solution is washed three times with 150 ml of saturated brine, dried by anhydrous sodium sulfate, and then evaporated to remove the solvent, and the residue is purified by column using DCM/PE to obtain compound 12-3. The yield is about 60%.
2) Synthesis of compound 12: raw material 12-3 (10.8 g, 20 mmol) and titanium tetrachloride (0.228 g, 1.2 mmol) are dissolved in 100 ml of chloroform under protection of nitrogen, then bis(trimethylsilyl)carbodiimide (11.2 g, 60 mmol) is added under an ice bath, and the reaction solution is stirred overnight at room temperature. The compound 12 is obtained after washed with water, dried, and purified by column chromatography, and MS:[M+H]+=588.4. The yield is 57.1%.

2. Manufacture and characteristics of OLED devices.

A device structure of ITO/HIL(10nm)/HTL(50nm)/host:10% dopant(40nm)/ETL:Liq(30nm)/Liq(lnm)/Al(100nm)/cathode has following manufacturing steps. a. Cleaning of conductive glass substrates: when used for the first time, they may be cleaned with various solvents, such as chloroform, ketone, and isopropanol, and then subjected to ultraviolet ozone plasma treatment.
b. HII,(10nm), HTL(50nm), EML(40nm), and ETL(30nm) may be formed by thermal evaporation in high vacuum (1 × 10-6 mbar).
c. Cathode: Liq/Al(lnm/100nm) may be formed by thermal evaporation in high vacuum (1×10-6 mbar).
d. Encapsulation: devices are encapsulated with UV-curable resins in the glove box under the nitrogen atmosphere.

Wherein, an EML material is the host: 10% dopant(40nm), and an ETL material is ETL: Liq(30nm).

Wherein, HIL is compound 1-12 and comparative compound 1-2.

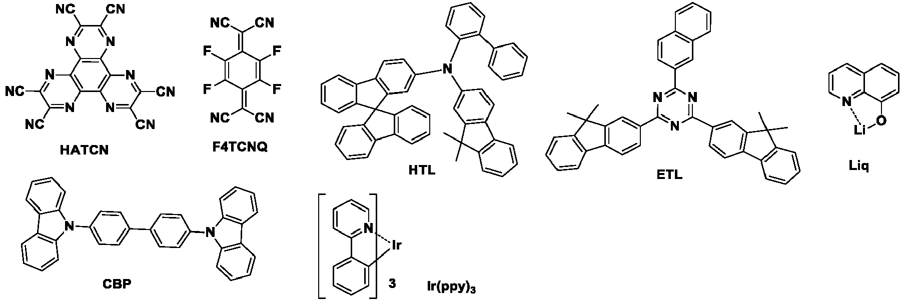

Comparative compound 1 is F4TCNQ.

Comparative compound 2 is HATCN.

Current-voltage (J-V) characteristics of each OLED device are characterized by a characterization equipment while recording important parameters such as efficiency, service life, and external quantum efficiency. After tested, the efficiency and service life of the devices obtained by using compounds 1 to 10 as HIL are better than those of the comparative examples, as shown in Table 1.

**Table 1**

| device example | HIL | Voltage (V) | Efficiency (cd/A) | Service life (LT95, h) @1000cd/m² |
|---|---|---|---|---|
| device example 1 | compound 1 | 3.6 | 70 | 13000 |
| device example 2 | compound 2 | 3.7 | 76 | 13500 |
| device example 3 | compound 3 | 3.6 | 76 | 13100 |
| device example 4 | compound 4 | 3.6 | 75 | 13000 |
| device example 5 | compound 5 | 3.6 | 78 | 13700 |
| device example 6 | compound 6 | 3.6 | 78 | 13500 |
| device example 7 | compound 7 | 3.6 | 78 | 13000 |
| device example 8 | compound 8 | 3.6 | 78 | 13800 |
| device example 9 | compound 9 | 3.6 | 80 | 13500 |
| device example 10 | compound 10 | 3.6 | 80 | 13000 |
| device example 11 | compound 11 | 3.6 | 80 | 13800 |
| device example 12 | compound 12 | 3.6 | 75 | 12900 |
| comparative example 1 | comparative compound 1 | 3.8 | 60 | 6670 |
| comparative example 2 | comparative compound 2 | 3.8 | 60 | 6700 |

From table 1, it can be known that using the compound of the present disclosure as a material of the hole injection layer of the organic electronic device can greatly improve the efficiency and service life of the organic electronic device when compared to comparative compounds 1 and 2. Particularly, compared to the comparative examples, the service life of the device example 11 is increased by about two times, and the efficiency thereof is increased by nearly 33%.

The technical features of the above-described embodiments can be combined arbitrarily. For the sake of brevity, all possible combinations of the technical features in the above-described embodiments are not described. However, as long as there is no contradiction between the combinations of these technical features, all should be regarded as the scope described in the present disclosure.

The above-mentioned embodiments only express several implementations of the present disclosure, and the description thereof is more specific and detailed, but cannot be understood as a limitation on the scope of the present disclosure. It should be noted that for those having ordinary skills in the art, without departing from the concept of the present disclosure, several modifications and improvements can be made, and these all belong to the protection scope of the present disclosure. Therefore, the protection scope of the present disclosure shall be subject to the appended claims.

## Claims

1. A compound used for an organic electronic device, **characterized in that** the compound has a structure as shown by general formula (1): 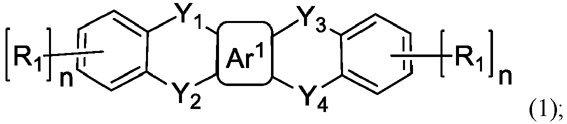 wherein Ar¹ is selected from structural formulas (A-1) or (A-2): 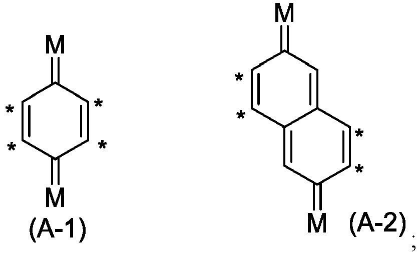
M is selected from CR₂R₃, NR₂, SiR₂R₃, PR₂, a substituted or unsubstituted aromatic group with 6 to 60 carbon atoms, a substituted or unsubstituted heteroaromatic group with 5 to 60 ring atoms, or a non-aromatic ring group with 3 to 30 ring atoms;
Y₁ to Y₄ are each independently selected from a single bond, CR₄R₅, NR₄, O, S, SiR₄R₅, PR₄, P(=O)R₄, S=O, S(=O)₂, or C=O; and Y₁ and Y₂ are not single bonds at a same time, and Y₃ and Y₄ are not single bonds at a same time;
n is an integer ranging from 0 to 4;
R₁ to R₅ are independently selected from H, D, a linear alkyl group, alkoxy group, or thioalkoxy group with 1 to 20 carbon atoms, a branched alkyl group, cyclic alkyl group, alkoxy group, or thioalkoxy group with 3 to 20 carbon atoms, a silyl group, a keto group with 1 to 20 carbon atoms, an alkoxycarbonyl group with 2 to 20 carbon atoms, an aryloxycarbonyl group with 7 to 20 carbon atoms, a cyano group, a carbamoyl group, a haloformyl group, a formyl group, an isocyano group, an isocyanate group, a thiocyanate group, an isothiocyanate group, a hydroxyl group, a nitro group, a nitroso group, CF₃, Cl, Br, F, I, a crosslinkable group, a substituted or unsubstituted aromatic group or heteroaromatic group with 5 to 60 ring atoms, an aryloxy group or heteroaryloxy group with 5 to 60 ring atoms, or a combination thereof; and adjacent R₁ are optionally bonded to each other to form a substituted or unsubstituted ring; and
^{∗} represents a linkage site.

2. The compound used for the organic electronic device according to claim 1, **characterized in that** the compound has a structure selected from general formulas (2-1) to (2-4):

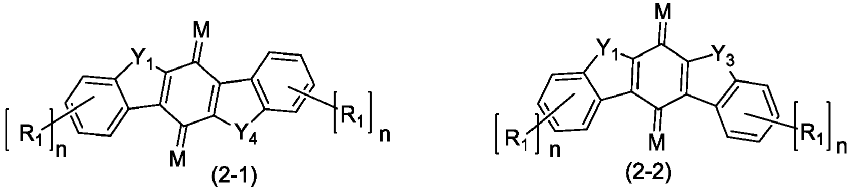

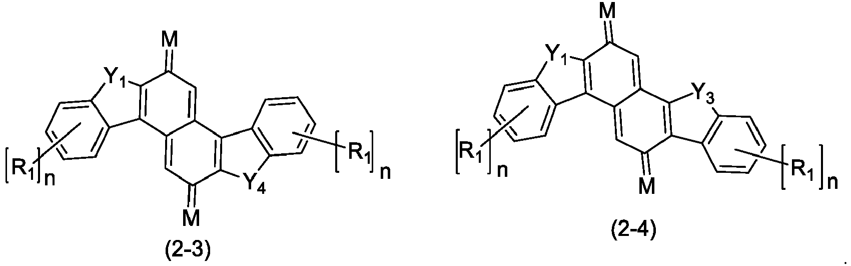

3. The compound used for the organic electronic device according to claim 2, **characterized in that** each R₁ is independently selected from a cyano group, a nitro group, a nitroso group, CF₃, Cl, Br, I, F, or an aromatic group or heteroaromatic group substituted with a cyano group, a nitro group, a nitroso group, CF₃, Cl, Br, I, or F.

4. The compound used for the organic electronic device according to claim 2, **characterized in that** in the general formulas (2-1) and (2-2), n is an integer ranging from 1 to 4.

5. The compound used for the organic electronic device according to claim 4, **characterized in that** each R₁ is independently selected from an aromatic group or heteroaromatic group substituted with a cyano group, a nitro group, a nitroso group, CF₃, Cl, Br, I, or F.

6. The compound used for the organic electronic device according to claim 2, **characterized in that** in the general formula (2-4), Y₁ and Y₃ are S.

7. The compound used for the organic electronic device according to claim 3 or 5, **characterized in that** the aromatic group or heteroaromatic group substituted with the cyano group, the nitro group, the nitroso group, CF₃, Cl, Br, I, or F is one selected from following groups: 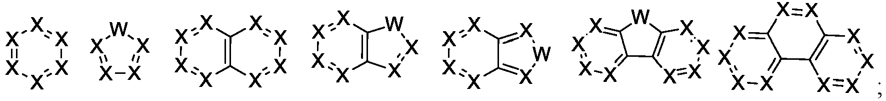
wherein, each X is independently selected from CR₇ or N;
each W is independently selected from CR₈R₉, NR₈, O, S, SiR₈R₉, PR₈, P(=O)R₈, S=O, S(=O)₂, or C=O; and
R₇ to R₉ are each independently selected from H, D, a linear alkyl group with 1 to 20 carbon atoms, a branched or cyclic alkyl group with 3 to 20 carbon atoms, a keto group with 1 to 20 carbon atoms, an alkoxycarbonyl group with 2 to 20 carbon atoms, an aryloxycarbonyl group with 7 to 20 carbon atoms, a cyano group, a carbamoyl group, a cyano group, a nitro group, a nitroso group, CF₃, Cl, Br, I, F, a substituted or unsubstituted aromatic or heteroaromatic group with 5 to 60 ring atoms, an aryloxy group or heteroaryloxy group with 5 to 60 ring atoms, or a combination thereof; and at least one R₇ is selected from a cyano group, a nitro group, a nitroso group, CF₃, Cl, Br, I, or F.

8. The compound used for the organic electronic device according to claim 7, **characterized in that** each R₁ is independently selected from 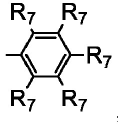 , wherein each R₇ is independently selected from H, D, a cyano group, a nitro group, a nitroso group, CF₃, Cl, Br, I, or F, and at least one R₇ is selected from a cyano group, a nitro group, a nitroso group, CF₃, Cl, Br, I, or F.

9. The compound used for the organic electronic device according to claim 1, **characterized in that** Ar¹ is selected from a structural formula (A-2).

10. The compound used for the organic electronic device according to claim 1, **characterized in that** M is selected from CR₂R₃ or NR₂.

11. The compound used for the organic electronic device according to claim 10, **characterized in that** M is one selected from following groups: 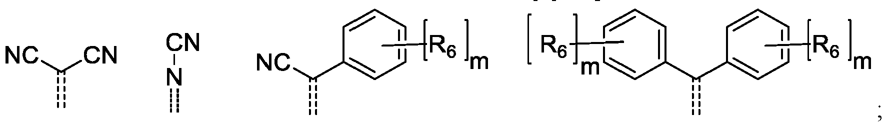
wherein each R₆ is independently selected from a cyano group, a nitro group, a nitroso group, CF₃, Cl, Br, I, or F; and
m is an integer ranging from 0 to 5.

12. The compound used for the organic electronic device according to claim 11, **characterized in that** M is one selected from following groups:

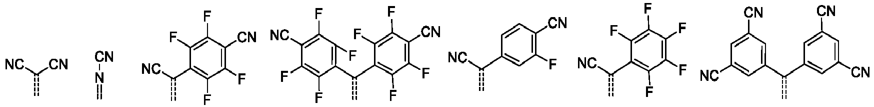

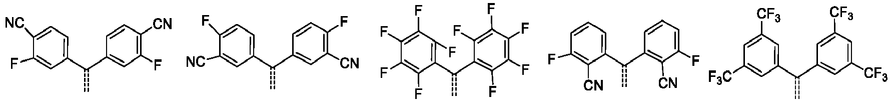

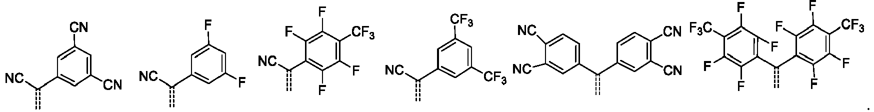

13. A mixture, **characterized in that** the mixture comprises the compound used for the organic electronic device according to one of claims 1 to 12 and at least one organic functional material, wherein the organic functional material is a hole injection material, a hole transport material, an electron transport material, an electron injection material, an electron blocking material, a hole blocking material, an emitter, a host material, or an organic dye.

14. A composition, **characterized in that** the composition comprises the compound used for the organic electronic device according to one of claims 1 to 12 or the mixture according to claim 13, and at least one organic solvent.

15. An organic electronic device, **characterized in that** the organic electronic device comprises at least one functional layer, and a material of the functional layer comprises the compound used for the organic electronic device according to one of claims 1 to 12 or the mixture according to claim 13, or is prepared by the composition according to claim 14.

16. The organic electronic device according to claim 15, **characterized in that** the functional layer is a hole injection layer.

17. The organic electronic device according to claim 15, **characterized in that** the organic electronic device is an organic light-emitting diode, an organic photovoltaic cell, an organic light-emitting cell, an organic field effect transistor, an organic laser, an organic spintronic device, an organic sensor, or an organic plasmon-emitting diode.
